# EUROPEAN PATENT SPECIFICATION

(11) **EP 3 160 342 B1**
(45) Date of publication and mention of the grant of the patent: **21.08.2024**
(21) Application number: 15810945.4
(22) Date of filing: 25.06.2015
(51) Int. Cl.: A61B 5/08

(54) **PLEURAL AIR LEAK TEST SYSTEM**
SYSTEM ZUM TESTEN AUF PLEURALLUFTLECKS
SYSTÈME DE CONTRÔLE DE FUITE D'AIR PLEURAL

(30) Priority: 25.06.2014 US 201462016898 P
(43) Date of publication of application: 03.05.2017
(73) Proprietor: Neomend, Inc., Irvine, California 92618 (US)
(72) Inventor: DAVIS, Peter, G., Dana Point, CA 92629 (US)
(74) Representative: Murgitroyd & Company
(86) International application number: PCT/US2015/037601
(87) International publication number: WO 2015/200582

(56) References cited:
- WO-A1-2013/123338
- GB-A- 2 306 891
- US-A1- 2005 016 542
- US-A1- 2007 251 532
- US-A1- 2010 174 270
- US-A1- 2013 245 484

## Description

### FIELD

Disclosed are methods, apparatus, and systems useful for detecting leaks in pleural tissue. Also disclosed are methods of detecting and sealing such leaks.

### BACKGROUND

Detecting the location of a pleural air leak is a difficult task. Current methods include high resolution cat scans, MRIs, and bronchoscopy for direct visualization of proximal airway leaks and bronchopleural fistulas. These methods offer varying levels of reliability and satisfaction but are not consistently sensitive and accurate, and require the use of costly and time intensive apparatus, and may require that the patient be moved. One of the most definitive means to identify a pleural air leak is through the relatively crude technique of open thoracotomy, in which the chest cavity is opened and filled with saline solution and then, following positive pressure ventilation of the lung, the observation of bubble formation indicates air leakage and directionally points to the area of leakage.

Alternatively, this method may be performed by submerging portions of or the entire lung in saline and observing bubbles as an indicator for a leak.

Often in current surgical practice a surgeon does not perform a leak test at all, for many reasons, including but not limited to time demands, the physical manipulation needed to submerge the lung, the difficulty observing leaks on the posterior lung, and the difficulty in tracking an air bubble to its origin, difficulty in recognizing or marking the location for later treatment, etc.

Thus, among other things, it would be beneficial to provide a visual identifier for an air leak pathway or pathways. US2007/0251532 A2 discloses a system and method for detection and repair of pulmonary air leaks.

### SUMMARY OF INVENTION

The invention is as defined in claims 1 to 3.

Disclosed but not claimed is a method of identifying and sealing an air leak in lung tissue in a patient in need thereof, the method comprising introducing a first composition comprising an indicator and a first component of a multi-part sealant into the lung via an aerosol or nebulizer, allowing the first composition to accumulate at one or more air leak, applying a second composition comprising a second component of a multi-part sealant externally to the lung at locations where the indicator has accumulated, and allowing the first component and the second component of the multi-part sealant to cure with one another to form a pleural sealant.

Disclosed is where the first composition is introduced to the patient in need thereof via the breathing apparatus.

Disclosed but not claimed is a method of identifying and sealing an air leak in lung tissue in a patient in need thereof, the method comprising introducing an indicator compound into the lung via an aerosol or nebulizer, allowing the indicator compound to accumulate at one or more air leak, applying a suture or sealant at a location identified by accumulated indicator compound.

Disclosed is a pressurized canister for facilitating identification of pleural air leaks, the pressurized canister comprising a canister capable of holding a pressurized fluid; a pressurized fluid comprising one or more indicator compounds.

Some embodiments provide a pleural air leak detection kit, the kit comprising a pressurized canister for facilitating identification of pleural air leaks, the pressurized canister comprising a canister capable of holding a pressurized fluid, a pressurized fluid comprising one or more indicator compounds; and a dispenser apparatus for housing and actuating the pressurized canister, wherein the dispenser apparatus has a dispensing end capable of coupling to a breathing apparatus to permit flow of indicator compound into the lungs via the breathing apparatus.

In some embodiments the dispenser is adapted for coupling to a breathing tube.

These and other variants will be readily apparent to one of skill in the art in light of the present disclosure. The description herein is meant to be exemplary in nature.

### BRIEF DESCRIPTION OF THE DRAWINGS

Fig. 1 depicts a two different introduction apparatus, a syringe and an aerosolizer both adapted to receive a pressurized canister containing the indicator.
Fig. 2 depicts an exemplary system employing a syringe to introduce stain or dye into a tube leading to the lung.

### DETAILED DESCRIPTION

Identifying and marking one or more pleural air leaks can be easily and conveniently accomplished by introducing an identifier, such as a stain or dye, into the lung. Introduction into the lung is accomplished by aerosolizing the stain or dye so it may travel through the airway(s). The identifier can be introduced to the lung via the normal airways, for example via the trachea, either or both of the main bronchi, or if desired at some other location. Upon introduction, the stain will travel most readily into the path or paths of least resistance, which will correspond to paths terminating in an air leak. The identifier will localize in or through the pathways ending in an air leak. On the exterior surface of the lung, the identifier will become visible, as it colors the exposed tissue at the leak site. Without the identifier, the exposed tissue is nearly impossible to distinguish from surrounding tissue. Once the identifier works its way through the pathway, it will be readily visible at the surface, exposing the leak for subsequent treatment, such as sealing.

The identifier may be any suitable medically acceptable dye, stain, or other colorant. Appropriate identifiers are easily visible against lung tissue, are biocompatible, and inert. In some embodiments, the identifier should not interfere with bleeding and should be substantially free of clumping to prevent blockage of the airways to be identified. The identifier may include a stain, such as but not limited to methylene blue or indocyanine green or FD&C blue or other FD&C approved colorants. The blue or green color of these identifiers is particularly visible against the red and pink color of the lung tissue permitting rapid identification by means of direct visualization of the location of a leak.

In some embodiments, the identifier has a viscosity and particle size sufficient to allow delivery as an aerosol or via nebulizer. In some embodiments, the identifier is a flowable aerosolizable particle capable of delivery to the lung parenchyma. In some embodiments, the identifier may be or may include a surfactant that has a starting low viscosity and small particle size such that it may be nebulized and delivered via anesthesia endoscope to the lung. In most instances, the identifier is most easily delivered via an endoscope into the lung. This may be accomplished with limited additional equipment, since the indicator may be introduced and aerosolized directly into an already existing anesthesia endoscope or, for example, oxygen supply tube.

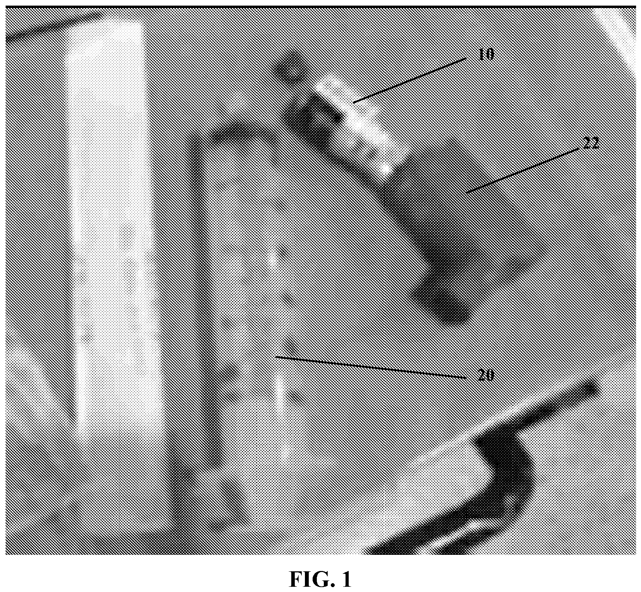

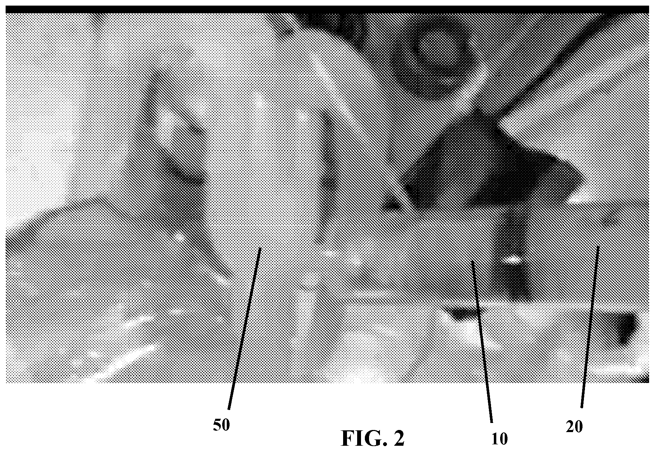

The identifier could be a stain that is introduced into the lung via a breathing tube where the stain is aerosolized and can be transferred into the lung. The ideal stain will travel along the path of least resistance and focalize in or through an air leak pathway of the bronchioles of the lung. The stain is biocompatible and inert. The stain would not interfere with bleeding and can be a color easily seen on tissue. The ideal stain could be provided in an inhaler like unit (Figs. 1, 2) that can be loaded into a section of the anesthesia equipment and expelled by pressure.

Once identified, the air leak can be addressed. In some uses of the claimed kit, a pleural sealant, such as a hydrogel sealant such as those marketed by NEOMEND under the PROGEL brand name may be applied to the exterior of the lung, wherever indicator is present. In this manner, the air leak may be sealed. Any acceptable pleural air sealant may be used. Further examples of such hydrogel sealants can be found in US patents: US Re38158; USRe38827; US8,409,605; US5,502,902; US5,856,367; US6,371,975; US6,458,147; US6,899,889; US7,247,314; US6,830,756; US7,318,933; US8,034,367; US6,458,095; and US6,569,113.

In some uses of the claimed kit, the indicator may be introduced into the lung at substantially the same time as a first component of a two part adhesive or sealant, such as those alluded to above. The indicator and first component are introduced as described above, via an aerosol or nebulizer, such that the indicator and first component are carried along the path or paths of least resistance to the air leak site or sites. In this manner, the first component of the adhesive or sealant is carried directly to the point of air leakage, along with the indicator. This, as above, is preferably done under pressure, which forces the indicator and first component to the air leak sites.

Because the indicator is visible on the surface of the lung at the air leak site or site, the leak sites are readily ascertainable, and a second component of the two component adhesive may be applied externally at those sites. This facilitates quick, consistent seal and avoids any possibility of inadvertent sealing within the lung. In some embodiments, the second component of the adhesive may also comprise a second indicator, such as a stain or dye. Upon application of the second component, the second indicator, which is different in color than the first and/or causes a color change, shows which leaks have been treated. For example, if the first component includes methylene blue as an indicator, the second component could include a yellow dye, such that the yellow either covers the blue or turns green when the two combine. In this manner, the surgeon is provided with immediate visual confirmation as to which air leaks have and have not been treated.

As described above, disclosed but not claimed is a method of identifying and sealing a pleural air leak in a patient in need thereof. The disclosed method comprises introducing an indicator and a first component of a multi-part sealant into the lung via an aerosol or nebulizer, allowing the indicator to accumulate at one or more air leak, applying a second component of a multi-part sealant externally to the lung at locations where the indicator has accumulated, and allowing the first component and the second component of the multi-part sealant to cure with one another to form a pleural sealant.

The multi-part sealant may be any suitable sealant. The multi-part sealant in some embodiments is readily formed from a two component mixture which includes a first part of a protein, preferably an albumin protein, such as human serum albumin or recombinant human albumin, in an aqueous buffer having a pH in the range of about 8.0-11.0 and a second part of a water-compatible or water-soluble bi-functional or multi-functional crosslinking agent or combinations thereof. When the two parts of the mixture are combined, the mixture is initially a liquid which cures *in vivo* on the surface of tissue in less than about one minute to give a strong, flexible, pliant substantive composition which bonds to the tissue and is absorbed in about four to sixty days. Particularly contemplated are two-part sealants which employ PEG and human albumin (natural or recombinant), such as those alluded to above.

To facilitate the introduction of the indicator, a pressurized canister containing the indicator, for example methylene blue, is provided. The canister is charged with a given amount of indicator at a given pressure sufficient to force the aerosolized indicator down a patient's breathing tube and into the airways. The breathing tube is managed by an anesthesiologist during the procedure which reduces the time the thoracic surgeon spends using saline to submerge the lung and looking for bubbles. Reduced surgical times are almost always beneficial to the patient. The clear presence of indicator on the surface of the lung at the point of air leakage provides a clear visual target for the surgeon, which in addition to saving time searching for leaks, allows for the opportunity to apply additional sutures or sealants with confidence. This should lead to better outcomes.

In some embodiments, the pressurized canister 10 containing the indicator may be a metered dose canister, similar to those used in an inhaler, which is capable of delivering a metered dose of the indicator. In other embodiments, the pressurized canister can be unmetered, with its contents released based upon user interaction. For example, the pressurized canister 10 can be placed in a delivery device such as a syringe 20, which is coupled to a patient's breathing tube 50, or other tube in communication with the lung or lungs, as seen in Figs. 1 and 2. The user, for example the anesthesiologist, can control the flow of indicator into the patient's lung or lungs by depressing the syringe plunger, which in turn compresses the pressurized canister trigger mechanism to release its contents. The indicator is aerosolized as it leaves the pressurized canister and forced into the breathing tube as a result of the release of the pressure. In metered embodiments, a pre-set amount of indicator is released. In un-metered embodiments, the indicator flows freely from the pressurized canister until the user releases or pull back on the plunger to stop the flow. The user may introduce 1 or more flows of indicator.

The pressurized canister may be charged with a composition which includes one or more indicator as described above, and one or more propellant. Any medically suitable propellant can be used. In some embodiments, the pressurized canister is sized for single use. By single use, it is meant a single surgical procedure. In some embodiments, the amount of indicator provided in a single canister is sufficient for testing the integrity of both lungs at least once. In some embodiments, a single canister may contain sufficient indicator for multiple tests during the same procedure.

Some embodiments provide a lung leak test kit including a pressurize canister housing an indicator, such as methylene blue or indocyanine green, a dispenser for housing the canister for fluid connection with a breathing tube, wherein the dispenser permits activation of the canister to release a metered amount of the indicator, or a free flow of the indicator.

## Claims

1. A kit to identify an air leak in lung tissue in a patient in need thereof, the kit comprising:
a pressurized canister (10) including a first composition comprising an indicator and a first component of a multi-part sealant,
a second composition to be applied externally to the lung, the second composition comprising a second component of the multi-part sealant,
and a dispenser apparatus (20) for housing and actuating the
pressurized canister, wherein the dispenser apparatus has a dispensing end capable of coupling to a breathing apparatus (50) to permit flow of the first composition into the lungs via the breathing apparatus.

2. The kit of claim 1, wherein the dispenser (20) is adapted for coupling to a breathing tube.

3. The kit of claim 1 or 2, wherein the second composition further comprises a second indicator, wherein the indicator in the second composition is a different color than the color of the indicator in the first composition.

## Patentansprüche

1. Ein Kit zur Identifizierung eines Luftlecks in Lungengewebe bei einem Patienten, der dieses benötigt, wobei das Kit Folgendes beinhaltet:
einen unter Druck stehenden Behälter (10), der eine erste Zusammensetzung umfasst,
die einen Indikator und eine erste Komponente eines mehrteiligen Dichtungsmittels beinhaltet,
eine zweite Zusammensetzung, die von außen her auf die Lunge aufzutragen ist, wobei die zweite Zusammensetzung eine zweite Komponente des mehrteiligen Dichtungsmittels beinhaltet,
und eine Ausgebervorrichtung (20) zur Aufnahme und Betätigung des unter Druck stehenden Behälters, wobei die Ausgebervorrichtung ein Ausgeberende aufweist, das mit einer Atemvorrichtung (50) gekoppelt werden kann, um einen Fluss der ersten Zusammensetzung über die Atemvorrichtung in die Lungen zu ermöglichen.

2. Kit gemäß Anspruch 1, wobei der Ausgeber (20) zur Kopplung mit einem Atemschlauch angepasst ist.

3. Kit gemäß Anspruch 1 oder 2, wobei die zweite Zusammensetzung ferner einen zweiten Indikator beinhaltet, wobei der Indikator in der zweiten Zusammensetzung eine Farbe aufweist, die sich von der Farbe des Indikators in der ersten Zusammensetzung unterscheidet.

## Revendications

1. Un kit pour identifier une fuite d'air dans du tissu pulmonaire chez un patient en ayant besoin, le kit comprenant :
une cartouche sous pression (10) incluant une première composition comprenant un indicateur et un premier constituant d'un agent d'étanchéité en plusieurs parties,
une deuxième composition à appliquer à l'extérieur du poumon, la deuxième composition comprenant un deuxième constituant de l'agent d'étanchéité en plusieurs parties,
et un appareil distributeur (20) pour accueillir et actionner la cartouche sous pression, l'appareil distributeur ayant une extrémité de distribution à même d'être accouplée à un appareil respiratoire (50) afin de rendre possible l'écoulement de la première composition jusque dans les poumons par le biais de l'appareil respiratoire.

2. Le kit de la revendication 1, dans lequel le distributeur (20) est conçu pour être accouplé à un tube raccord.

3. Le kit de la revendication 1 ou de la revendication 2, dans lequel la deuxième composition comprend en outre un deuxième indicateur, l'indicateur dans la deuxième composition étant d'une couleur différente de celle de l'indicateur de la première composition.
